# EUROPEAN PATENT SPECIFICATION

(11) **EP 2 240 569 B1**
(45) Date of publication and mention of the grant of the patent: **11.09.2013**
(21) Application number: 08851525.9
(22) Date of filing: 18.11.2008
(51) Int. Cl.: C12M 1/40, C12N 11/08, C12N 11/12, C12P 17/06

(54) **LOVASTATIN ESTERASE ENZYME IMMOBILIZED ON SOLID SUPPORT, PROCESS FOR ENZYME IMMOBILIZATION, BIOCATALYTIC FLOW REACTOR AND PROCESS FOR PREPARATION AND/OR PURIFICATION OF SIMVASTATIN**
AUF EINEM FESTEN TRÄGER IMMOBILISIERTES LOVASTATIN-ESTERASE-ENZYM, VERFAHREN ZUR ENZYMIMMOBILISIERUNG, BIOKATALYTISCHER DURCHFLUSSREAKTOR UND VERFAHREN ZUR HERSTELLUNG UND/ODER REINIGUNG VON SIMVASTATIN
ENZYME LOVASTATINE ESTÉRASE IMMOBILISÉE SUR UN SUPPORT SOLIDE, PROCÉDÉ D'IMMOBILISATION D'ENZYME, RÉACTEUR À CIRCULATION BIOCATALYTIQUE ET PROCÉDÉ DE PRÉPARATION ET/OU DE PURIFICATION DE LA SIMVASTATINE

(30) Priority: 19.11.2007 PL 38381907
(43) Date of publication of application: 20.10.2010
(73) Proprietor: Instytut Chemii Organicznej, Polska Akademia Nauk, 01-224 Warszawa (PL)
(72) Inventor: OSTASZEWSKI, Ryszard, PL-05-806 Pecice Male (PL); KOSZELEWSKI, Dominik, PL-02-784 Warszawa (PL); KUREK, Waldemar, PL-01-496 Warszawa (PL); PATRALSKA, Dorota, PL-08-110 Siedlce (PL)
(74) Representative: Pankowski, Jacek
(86) International application number: PCT/PL2008/000086
(87) International publication number: WO 2009/067032

(56) References cited:
- EP-A- 0 486 153
- WO-A-2005/040107
- US-A- 4 556 637
- BIOTECHNOLOGY: "The studies on immobilization of lovastatin esterase" BIOTECHNOLOGY, 2006, pages 888-892, XP009112187 Czech Republic cited in the application
- KUREK WALDEMAR ET AL: "Studies on isolation and purification lovastatin hydrolase" JOURNAL OF BIOTECHNOLOGY, vol. 118, no. Suppl. 1, August 2005 (2005-08), page S116, XP002514883 & 12TH EUROPEAN CONGRESS ON BIOTECHNOLOGY (ECB 12); COPENHAGEN, DENMARK; AUGUST 21 -24, 2005 ISSN: 0168-1656
- BLAIS B W ET AL: "ACTIVATION OF POLYESTER CLOTH FOR THE IMMOBILIZATION OF A POLYSACCHARIDE ANTIGEN" BIOTECHNOLOGY TECHNIQUES, vol. 4, no. 1, 1990, pages 11-14, XP002514884 ISSN: 0951-208X
- CHEN ET AL: "Production by Clonostachys compactiuscula of a lovastatin esterase that converts lovastatin to monacolin J" ENZYME AND MICROBIAL TECHNOLOGY, STONEHAM, MA, US, vol. 39, no. 5, 4 September 2006 (2006-09-04), pages 1051-1059, XP005561810 ISSN: 0141-0229
- SCHIMMEL TIMOTHY G ET AL: "Purification and characterization of a lovastatin esterase from Clonostachys compactiuscula" APPLIED AND ENVIRONMENTAL MICROBIOLOGY, vol. 63, no. 4, 1997, pages 1307-1311, XP002514885 ISSN: 0099-2240
- HAJJAJ H ET AL: "Lovastatin biosynthesis by Aspergillus terreus in a chemically defined medium." APPLIED AND ENVIRONMENTAL MICROBIOLOGY JUN 2001, vol. 67, no. 6, June 2001 (2001-06), pages 2596-2602, XP002514886 ISSN: 0099-2240 cited in the application

## Description

This invention relates to the lovastatin esterase enzyme immobilized on a solid support insoluble in water, a process for an enzyme immobilization, a biocatalytic flow reactor and a process for preparation and/or purification of simvastatin.

Simvastatin and lovastatin belong to the class of compounds being the hydroxy-3-methyl-glutaryl-coenzyme A (HMG-CoA) reductase inhibitors, named statins. Statins significantly reduce risk of coronary arterial disease, cerebral stroke and peripheral atherosclerosis. By reduction of cholesterol deposits, they stabilize the atheromatous plaque, improve the function of vascular endothelium, inhibit growth and migration of smooth muscle cells, and also have a beneficial effect on blood clotting, fibrinolysis, and the activity of platelets, and also exhibit an anti-inflammatory effect [Tobert J.A. et al., Journal of Clinical Investigations, 1982, April, 69 (4), 913-919; Pedersen T. et al., Lancet (1994) 344, 1383-89; *Czynniki Ryzyka. Kwartalnik Polskiego Towarzystwa Badań nad Miażdżyc* 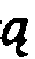 *(Risk Factors. Polish Society of Atherosclerosis Research Quarterly)* 2003, 40, 5-13].

Lovastatin, the compound of the formula I, is prepared *via* fermentation using the *Aspergillus terreus* strain. Simvastatin, the compound of the formula II, having a higher pharmacological activity and a lower toxicity than lovastatin, is prepared semi-synthetically from lovastatin.

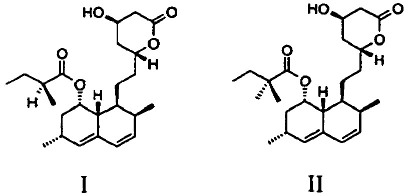

U.S. patent No. 4,582,915 discloses a process for preparing simvastatin from lovastatin. Lovastatin is subjected to the alkaline hydrolysis, and the formed potassium salt is treated with a strong base such as *n*-butyllithium in the presence of a secondary amine, followed by a methylating agent (e.g., methyl iodide), according to the following scheme.

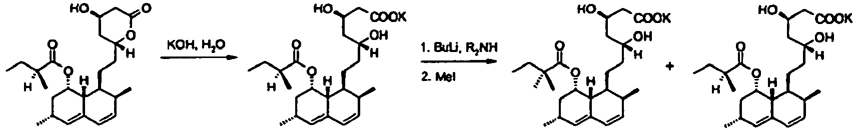

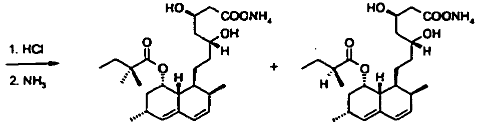

The resulting conversion does not exceed 95%. Isolation of simvastatin in its pure form is difficult because the contamination with residual lovastatin can be separated, in principle, only with the use of HPLC.

The European patent EP 0486153 discloses an enzymatic hydrolysis of lovastatin acid or a salt thereof using an enzyme derived from *Clonostachys compactiuscula,* to form a triol acid. Said triol acid may be converted in a straightforward manner to a lactone form (diol lactone). An immobilized enzyme column may be employed for *Clonostachys compactiuscula* enzyme.

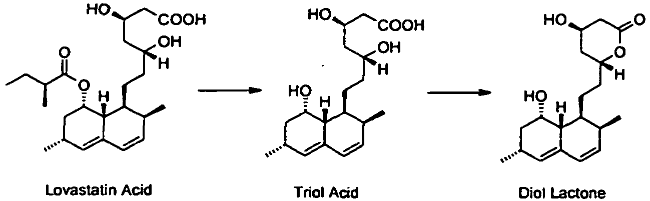

The international publication WO 2005/040107 discloses a method for making simvastatin, said method comprising an enzymatic hydrolysis of lovastatin, lovastatin acid or a salt of lovastatin acid, followed by a lactonization and regioselective acylation of the resulted triol acid, and further by a chemical or enzymatic acylation to form 4-acyl simvastatin. The selective removal of acyl group protecting 4-hydroxy group yields simvastatin.

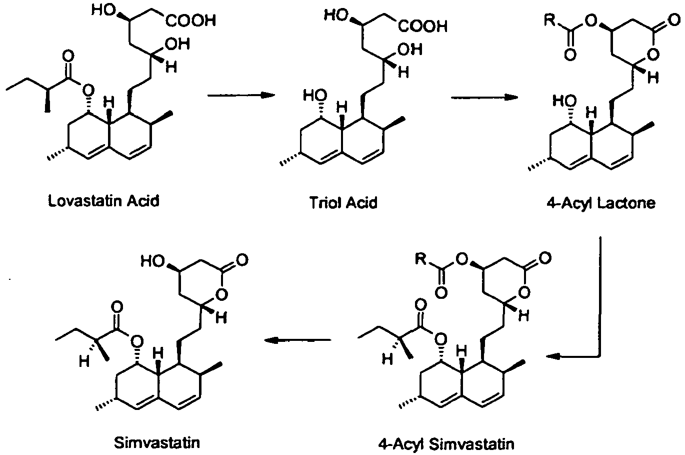

U.S. patent No. 4,556,637 relates to an immobilized cholinesterase enzyme, and a process for preparation of immobilized cholinesterase enzyme, wherein the polymeric resin is treated with a carbodiimide derivative to form an activated support, followed by applying a solution of cholinesterase enzyme to said activated support.

Blais B.W. et al., in Biotechnology techniques (vol. 4, No. 1, 1990, pp. 11-14) discloses an immobilization of a polysaccharide antigen on a polyester cloth using cyanuric chloride as a coupling agent. The segments of polyester cloth are soaked with a solution of cyanuric chloride in an aprotic organic solvent, washed with dioxane and borate buffer, and the activated cloth is coated with a polysaccharide antigen. The unreacted cyanuric chloride residue is blocked by incubating the segment cloth with ethanolamine buffer.

Kurek W. et al., presents the initial results (on 12th European Congress on Biotechnology, Denmark, August 21-24, 2005; abstract: Journal of Biotechnology, vol. 118, p. S116) of studies on a purification and immobilization of lovastatin hydrolase. Following C-methylation of lovastatin, simvastatin is separated from a lovastatin residue using fungi *Clonostachys compactiuscula.* Since the use of living microbials is disadvantageous due to a high cost and difficult product isolation from a reaction mixture, a separation and immobilization of an enzyme that hydrolyses lovastatin ammonium salt in a presence of simvastatin ammonium salt is proposed.

The full experimental details of a lovastatin esterase immobilization on a solid support are disclosed by Ostaszewski R. et al., Biotechnology (2006, 888-892). Using an enzyme produced by the fungus *Clonostachys compactiuscula* (ATCC 38009, ATCC 74178) an immobilized form of an enzyme is prepared by entrapment of said enzyme on calcium alginate beads and in sol-gel matrix. The material obtained exhibits a significant reduction of the selectivity of hydrolysing lovastatin *versus* simvastatin, i.e., the enzymatic process resulted in hydrolysis of both lovastatin and simvastatin. An immobilization on Eupergit® C (bearing reactive oxirane groups capable to form a covalent bonding with an enzyme molecule) leads to the immobilized enzyme covalently bound to said support, but of low selectivity, also. The attempts to prepare an immobilized enzyme covalently bound to a support activated by a coupling agent - divinyl sulfone - result in materials characterized by a complete loss of hydrolytic activity and selectivity.

The object of this invention is to provide the lovastatin esterase enzyme immobilized on a solid support insoluble in water, a process for an enzyme immobilization, a biocatalytic flow reactor as well a process for preparation and/or purification of simvastatin.

The lovastatin esterase enzyme immobilized on a solid support insoluble in water, said enzyme being covalently bound to a solid support activated with an at least difunctional coupling reagent, according to the invention is characterized in that the combination of solid support and at least difunctional coupling reagent is such, that the immobilized lovastatin esterase exhibits at least 5 times higher the hydrolytic activity towards lovastatin and salts thereof, in the presence of simvastatin and/or salts thereof, than towards simvastatin and salts thereof, wherein:
- the solid support is a modified polysaccharide comprising di-(C₁₋₆alkyl)amino-C₁₋₆alkylcellulose, and the at least difunctional reagent activating the solid support is cyanuric halide and/or cyanuric acid O-sulphonate, or
- the solid support is a modified silica gel, especially modified with amino-C₁₋₆ alkyl-tri(C₁₋₆ alkoxy)silane, and the at least difunctional reagent activating the solid support is cyanuric halide and/or cyanuric acid O-sulphonate, or
- the solid support is a polygalactoside and the at least difunctional reagent activating the solid support is a compound of the formula 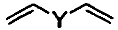 , in which Y represents -SO₂- or -SO₂-(CHR)ₙ-SO₂-, where n is an integer of from 1 to 18, and R is a hydrogen atom or C₁₋₆ alkyl, or Y represents -SO₂-Ar-SO₂-, where Ar is a divalent aryl radical formed by displacing two hydrogen atoms directly bound to the aromatic ring carbon atoms, the divalent aryl radical optionally bearing C₁₋₆ alkyl substituents.

Advantageously, the solid support is a polygalactoside, and the at least difunctional reagent activating the solid support is the compound of the formula , in which Y represents -SO₂-.

Advantageously, the solid support is diethylaminoethylcellulose, and the at least difunctional reagent activating the solid support is cyanuric chloride.

Advantageously, the solid support is an aminopropylsilanized silica gel, and the at least difunctional reagent activating the solid support is cyanuric chloride.

In particular, the enzyme is an enzyme produced by *Clonostachys compactiuscula* ATCC 38009, ATCC 74178.

A process for immobilization of the lovastatin esterase enzyme on a solid support insoluble in water, according to the invention, is characterized in that using mechanical agitation, in a solvent, a cyanuric halide is contacted with a solid support being modified polysaccharide comprising di-(C₁₋₆alkyl)amino-C₁₋₆alkylcellulose or a solid support being silica gel modified with amino-C₁₋₆alkyl-tri(C₁₋₆alkoxy)silane, the activated solid support is separated by filtration, then dried and suspended in an aqueous mixture containing the lovastatin esterase enzyme until immobilization of the enzyme, the suspended material is separated by filtration, washed with a buffer and dried.

In particular, the solid support is diethylaminoethylcellulose.

In particular, the solid support is an aminopropylsilanized silica gel.

Advantageously, the cyanuric halide used is cyanuric chloride.

In particular, an autoclaved solid support is used.

The enzyme-containing aqueous solution used is, especially, a protein fraction of the material extracted from *Clonostachys compactiuscula* ATCC 38009, ATCC 74178.

A process for immobilization of the lovastatin esterase enzyme on a solid support insoluble in water, according to the invention, is characterized in that the compound of the formula , in which Y represents -SO₂- or -SO₂-(CHR)ₙ-SO₂-, where n is an integer of from 1 to 18, and R is a hydrogen atom or C₁₋₆ alkyl, or Y represents -SO₂-Ar-SO₂-, where Ar is a divalent aryl radical formed by displacing two hydrogen atoms directly bound to the aromatic ring carbon atoms, the divalent aryl radical optionally bearing C₁₋₆ alkyl substituents, is contacted with the solid polygalactose support in a solvent using mechanical agitation, the activated solid support is separated by filtration, then dried and suspended in an aqueous mixture containing the lovastatin esterase enzyme until immobilization of the enzyme, the suspended material is separated by filtration, washed with a buffer and dried.

Advantageously, the compound of the formula 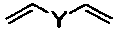 is a compound in which Y represents -SO₂-.

The enzyme-containing aqueous solution used is, especially, a protein fraction of the material extracted from *Clonostachys compactiuscula* ATCC 38009, ATCC 74178.

A biocatalytic flow reactor with a bed comprising a body of the reactor with an inner space connected to the fluid inlet and connected to the fluid outlet, in which inner space there is a bed containing the lovastatin esterase enzyme immobilized on a solid support insoluble in water, said enzyme being covalently bound to the solid support activated with an at least difunctional coupling reagent. according to the invention is characterized in that the combination of solid support and at least difunctional coupling reagent is such, that the immobilized lovastatin esterase exhibits at least 5 times higher the hydrolytic activity towards lovastatin and salts thereof in the presence of simvastatin and/or salts thereof, than towards simvastatin and salts thereof, wherein:
- the solid support is a polygalactoside, and the at least difunctional reagent activating the solid support is a compound of the formula , in which Y represents -SO₂-, or
- the solid support is diethylaminoethylcellulose, and the at least difunctional reagent activating the solid support is cyanuric chloride.

The enzyme is an enzyme produced, especially, by *Clonostachys compactiuscula* ATCC 38009, ATCC 74178.

A process for preparation and/or purification of simvastatin comprising treating the solution of the simvastatin salt containing residual content of the lovastatin salt with the lovastatin esterase enzyme until hydrolysing lovastatin to form the triol, separating the triol, and isolating simvastatin substantially free from lovastatin, where the solution of the simvastatin salt containing residual content of the lovastatin salt, is brought into a contact with the lovastatin esterase enzyme immobilized on a solid support insoluble in water, said enzyme being covalently bound to the solid support activated with an at least difunctional coupling reagent, according to the invention is characterized in that the combination of solid support and at least difunctional coupling reagent is such, that the immobilized lovastatin esterase exhibits at least 5 times higher the hydrolytic activity towards lovastatin and salts thereof in the presence of simvastatin and/or salts thereof, than towards simvastatin and salts thereof, wherein:
- the solid support is a polygalactoside, and the at least difunctional reagent activating the solid support is the compound of the formula , in which Y represents -SO₂-, or
- the solid support is diethylaminoethylcellulose, and the at least difunctional reagent activating the solid support is cyanuric chloride, or
- the solid support is an aminopropylsilanized silica gel, and the at least difunctional reagent activating the solid support is cyanuric chloride.

In particular, the enzyme is an enzyme produced by *Clonostachys compactiuscula* ATCC 38009, ATCC 74178.

Immobilization of the enzyme makes it possible to repeatedly reuse of the same enzyme without loss of its activity at the appropriately adjusted parameters of the process. What is extremely important, a resistance to the proteolytic degradation is enhanced, allowing to perform the process without using antiseptic conditions, thus lowering the costs significantly.

According to one embodiment of the invention, the immobilized enzyme is contained in a flow reactor. The reactor is supplied with a mixture of the statins comprising 15% lovastatin and 85% simvastatin. This is a typical mixture obtained in the chemical synthesis process. The concentration of the simvastatin salt does not change by more than 1%, whereas the lovastatin ammonium salt is hydrolysed in about 87%. The conversion level does not alter during the prolonged hydrolysis, and the technological stability is maintained for at least 6 months. Thus, the immobilization of the lovastatin esterase enzyme provides a stable biocatalyst exhibiting a stability sufficient for the technological application in the synthesis of simvastatin.

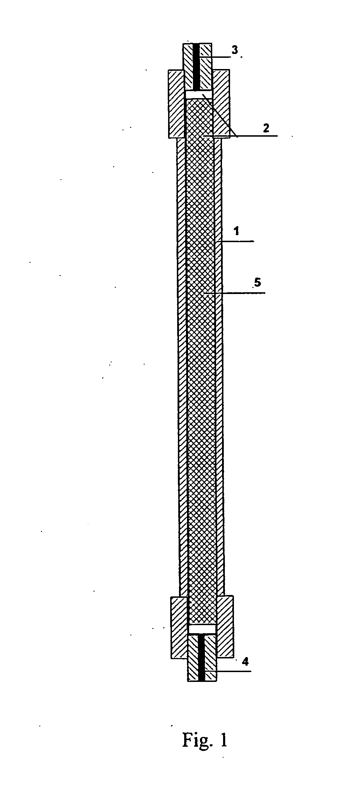

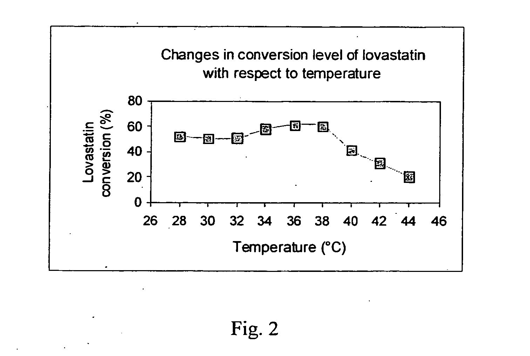

The technical solution according to the invention is illustrated in drawings, in which Fig. 1 presents a cross-sectional view of the biocatalytic flow reactor according to the invention, and Fig. 2 presents changes in the conversion level of lovastatin with respect to temperature.

Throughout the description of this invention and the patent claims, the term "triol" denotes the compound of the formula III, 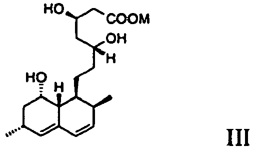 wherein M represents a hydrogen atom, and also wherein M represents a metal cation or ammonium cation, i.e., the compound in the free-acid form or in the salt form, respectively, if not specified otherwise. Since the compound III in the free-acid form (M=H) is easily lactonized, the term "triol" may also comprise the lactone-diol form of the formula IV, if not specified otherwise.

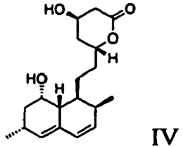

The names "lovastatin" and "simvastatin" refer to the compounds of the formulae I and II, respectively.

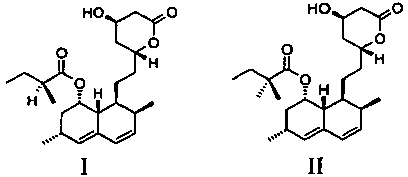

Throughout the description of this invention and the patent claims, the names "lovastatin" and "simvastatin" comprise also the carboxylic acid forms of these compounds, having the formulae IV and V (M=H), respectively, 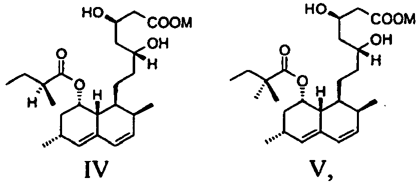 and also salts of the compounds of the formulae IV and V, if not specified otherwise. The "lovastatin salt" represents the compound of the formula IV, wherein M represents a metal cation or an ammonium cation, and the "simvastatin salt" represents the compound of the formula V, wherein M represents a metal cation or an ammonium cation.

Throughout the description of this invention and the patent claims, the "lovastatin esterase" denotes a cellular or non-cellular material of the natural or recombinant origin having an enzymatic activity that consists in catalysing the hydrolysis of lovastatin and salts thereof, to form the above-defined triol or salts thereof, where, under analogous conditions, the simvastatin salts do not undergo enzymatic hydrolysis or undergo enzymatic hydrolysis at a rate lower of at least one order of magnitude.

Throughout the description of this invention and the patent claims, the solid support insoluble in water denotes a granular or fibrous solid support, that principally does not undergo solubilisation in water, i.e., it does not form a liquid solution or pseudo-solution in water containing more than 0.1 g of the support per 100 g of water.

Throughout the description of this invention and the patent claims, the enzyme activity represents a micromolar amount of the substrate (lovastatin or simvastatin) that is hydrolysed within one minute with one milligram of the enzyme.

Throughout the description of this invention and the patent claims, the protein fraction represents a portion of the mixture obtained by purifying the biological preparation, in which portion the determined total concentration of proteins is greater than zero.

The hydrolytic activity of the lovastatin esterase enzyme towards lovastatin and salts thereof represents a capability of hydrolysing lovastatin and salts thereof to form the above-defined triol.

The hydrolytic activity of the lovastatin esterase enzyme, towards of simvastatin and salts thereof represents a capability of hydrolysing simvastatin and salts thereof to form the above-defined triol.

The hydrolytic activity of the lovastatin esterase enzyme towards lovastatin and salts thereof is at least by one order of magnitude higher than the hydrolytic activity of the lovastatin esterase enzyme towards simvastatin and salts thereof. This activity may be altered following immobilization of the lovastatin esterase enzyme on a solid support, particularly on a solid support insoluble in water. Without limiting the scope of the invention by theoretical considerations, one may suppose that because of chemical binding the above-defined enzyme at the surface of the solid phase, modifications of the spatial configuration of the enzyme may occur, thus altering the relative localization of the enzyme active sites. Because of that, the hydrolytic activity of the lovastatin esterase enzyme, as well as the enzyme activity following immobilization on a solid support may differ from the activity of the enzyme in an aqueous mixture. Such a difference manifests itself in a loss of selectivity of the hydrolytic activity following immobilization on a solid support and/or in decreasing the activity.

It was unexpectedly found that the particular combination of the solid support insoluble in water and the at least difunctional coupling reagent allows obtaining the enzyme immobilized on a solid support insoluble in water, that exhibits the hydrolytic activity towards lovastatin and salts thereof at least 5 times higher, in the presence of simvastatin and/or salts thereof, than towards simvastatin and salts thereof, where the enzyme is sufficiently active to be applied for purifying and/or isolating simvastatin from the mixture with lovastatin.

By the process according to the invention, the lovastatin esterase enzyme is immobilized on the polygalactose bed, such as agarose (preferably Sepharose B4), using the at least difunctional reagent of the formula 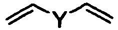 , in which Y represents -SO₂-or -SO₂-(CHR)ₙ-SO₂-, where n represents an integer of from 1 to 18, and R is a hydrogen atom or C₁₋₆ alkyl, or Y represents -SO₂-Ar-SO₂-, where Ar is a divalent aryl radical formed by displacing two hydrogen atoms directly bound to the aromatic ring carbon atoms, the divalent aryl radical optionally bearing C₁₋₆ alkyl substituents. Advantageously, the reagent activating the solid support is the compound of the formula 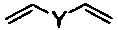 , wherein Y represents -SO₂-, i.e., divinylsulphone.

The process of activation of the polygalactose solid support, according to the invention, consists in contacting the support with the compound of the formula 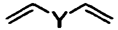 , preferably divinylsulphone, what results in formation of the activated solid support. Then the mixture containing the lovastatin esterase enzyme, prepared according to the known procedure, using the buffer, is contacted with the activated solid support with mechanical agitation, over a period sufficient to immobilize the enzyme on a solid support. Following the immobilization, the obtained biocatalyst is separated by filtration, preferably washed with water and/or a buffer. The immobilized enzyme according to the invention is then used in the processes of purification and/or isolation of simvastatin.

The enzyme immobilized on a solid support using divinylsulphone as a coupling reagent was used in the hydrolysis reaction of a mixture of statins containing 15% lovastatin and 85% simvastatin. This is a typical mixture obtained in the process for preparing simvastatin from lovastatin via the chemical synthesis. The flow reactor presented in the cross-sectional view in Fig. 1 has been designed and manufactured for carrying out the reaction. The flow reactor according to the invention comprises a body 1 of the reactor with an inner space 2 connected to a fluid inlet 3 and connected to a fluid outlet 4. The inner space 2 is filled with the bed 5 containing the immobilized enzyme. The hydrolysis process was carried out continuously by feeding the solution of a mixture of the ammonium salts of lovastatin and simvastatin via the fluid inlet 3 and receiving the efflux from the fluid outlet 4. The consecutive portions of the efflux were analysed by HPLC, proving that the concentration of the simvastatin salt was not altered during the process more than by 1%, with relation to the initial concentration. The degree of hydrolysis of the lovastatin ammonium salt was at least 87%. The conversion level was not altered during the process conducted continuously for 120 hours, what proved that the flow reactor according to the invention, with the bed 5 containing the immobilized lovastatin esterase enzyme, could be effectively used in the process of manufacturing, isolating and/or purifying simvastatin from the mixture of statins containing simvastatin and lovastatin.

The technological stability of the reactor according to the invention was examined by repeating the experiment concerning the hydrolysis of the mixture of statins containing 15% lovastatin and 85% simvastatin, after a 6-month storage of the reactor at +4°C. The hydrolysis process was conducted continuously by feeding the solution of a mixture of the ammonium salts of lovastatin and simvastatin via the fluid inlet 3, and receiving the efflux from the fluid outlet 4. The consecutive portions of the efflux were analysed by HPLC, proving that the concentration of the simvastatin salt was not altered during the process more than by 1%, with relation to the initial concentration. The degree of hydrolysis of the lovastatin ammonium salt was at least 96%. The data presented in the table 3 show that the storage time of the flow reactor does not influence significantly on its performance. Within 25 hours, the conversion of lovastatin was practically unchanged, what proved that the immobilized enzyme according to the invention retained its activity as well as the selectivity of hydrolysis of the lovastatin salts in the presence of the simvastatin salts. Therefore, immobilization of the enzyme on a solid support provides a stable biocatalyst having the stability sufficient for using in the process of manufacturing, isolating and/or purifying simvastatin.

In another embodiment of the process for immobilization of the enzyme on a solid support, according to the invention, the solid support insoluble in water, being a silica gel modified with an amino-C₁₋₆alkyl-tri(C₁₋₆alkoxy)silane, preferably a silica gel modified with aminopropylsilyl groups, was activated. The at least difunctional activating reagent, being a derivative of cyanuric acid (1,3,5-triazine-2,4,6-triol derivative), such as a cyanuric halide, or wherein at least two hydroxy groups were replaced with a halogen substituent or an O-sulphonate group, such as a cyanuric halide and/or cyanuric acid O-sulphonate, was used for the activation.

The silica gel modified with an amino-C₁₋₆alkyl-tri(C₁₋₆alkoxy)silane, was activated by the treatment with a solution of the derivative of cyanuric acid, followed by suspending in a buffer and adding the mixture containing the lovastatin esterase enzyme, with mechanical agitation, over a period sufficient to immobilize the enzyme on a solid support. The immobilization was conducted using the non-purified enzyme as well as the enzyme purified by chromatography. By using aminopropylsilanized silica gel as the preferred solid support and cyanuric chloride as the preferred coupling reagent, it was found that the use of the purified enzyme affords a higher efficiency of an enzyme immobilization. Moreover, the immobilized enzyme according to the invention, that is obtained using the non-purified enzyme, exhibits an activity lower than the immobilized enzyme according to the invention, that is obtained using the purified enzyme.

In another embodiment of the process for immobilization of the enzyme according to the invention, the solid support insoluble in water, such as the cellulose modified with di-(C₁₋₆alkyl)amino-C₁₋₆alkyl groups, is activated. An example of such a solid support is diethylaminoethylcellulose. The at least difunctional activating reagent, that is preferably a derivative of cyanuric acid (1,3,5-triazine-2,4,6-triol derivative), wherein at least two hydroxy groups are replaced with a halogen substituent or an O-sulphonate group, such as a cyanuric halide and/or cyanuric acid O-sulphonate, is used for activation.

The cellulose modified with di-(C₁₋₆alkyl)amino-C₁₋₆alkyl groups was activated using a solution of the derivative of cyanuric acid, optionally in the presence of a base, to yield the activated solid support. The solid support was suspended in a buffer and the mixture containing the lovastatin esterase enzyme was added, with mechanical agitation, over a period sufficient to immobilize the enzyme on a solid support. The immobilized enzyme according to the invention is then used in the processes of preparation, isolation and/or purification of simvastatin.

The enzyme immobilized on cellulose modified with diethylaminoethyl groups, using cyanuric chloride as a coupling reagent, was used in the hydrolysis of a mixture of statins, a mixture of ammonium salts of lovastatin and simvastatin, to give a very high conversion (>99%) of the lovastatin salt into the triol within several hours. The enzyme immobilized on a solid support diethylaminoethylcellulose, having even higher activity, was obtained by using the activated solid support diethylaminoethylcellulose, that was previously annealed in an autoclave.

The obtained immobilized enzyme according to the invention was used as a bed for the reactor according to the invention, as presented in Fig. 1. The reactor was placed in a thermostatted jacket having the temperature stabilized at 28°C. The process of hydrolysis was carried out continuously supplying the solution of a mixture of lovastatin and simvastatin ammonium salts via the fluid inlet 3 and collecting the efflux from the fluid outlet 4. The consecutive portions of the efflux were analysed by HPLC every 4 hours. During the operation of the reactor, the flow rate was modified and the lovastatin conversion changes were recorded. It was found that a reduction of the conversion of lovastatin occurs for the flow rates exceeding the limiting flow rate (*i.e*., the maximum flow rate at which the conversion of lovastatin is 100%). For the flow rates higher than the limiting flow rate (when the conversions are significantly lower than at the limiting flow rate), a change of the conversion of lovastatin with respect to temperature of the reactor was determined. The measurements were taken for a series of temperatures. The results are presented in the diagram (Fig. 2).

At 37°C, the lovastatin esterase enzyme immobilized on a solid support insoluble in water according to the invention was found to exhibit the highest activity (Fig. 2).

The technical solution according to the invention is presented in more detail by the following examples. Throughout the following examples, the term the "LE enzyme" represents the lovastatin esterase enzyme.

### EXAMPLES

### Example 1. Isolation and purification of the enzyme

The LE enzyme is produced by the fungus *Clonostachys compactiuscula* deposited under the number ATCC 38009. The fungus was harvested according to the procedure described in the literature (U.S. patent No. 5,223,415).

### 1a. Extraction of the LE enzyme from the mycelium

The mycelium of *Clonostachys compactiuscula* (59 g) was triturated with glass beads (0.4 g) in liquid nitrogen for 8 hours, then it was extracted with the phosphate buffer (60 mL, pH=6.5), and the solid was centrifuged off (12000 rpm; 10 min; 4°C). The supernatant was separated and the extraction procedure with buffer (40 mL) was repeated. The supernatants were combined and filtered *via* a qualitative filter to yield 110 mL of the filtrate, denoted as the supernatant X1 (Cₚᵣₒₜₑᵢₙ=4180 µg/mL) in the following description.

### 1b. Purification of the lovastatin esterase by salting out the active protein fraction

To the supernatant X1 (20 mL), ammonium sulphate was added to achieve the concentration of 40% (4.62 g, 1 hour, 0°C). The solution was then left for 1 hour (0°C), and centrifuged (12000 rpm; 20 min; 4°C). The supernatant was separated and ammonium sulphate was added to achieve the concentration of 85% (6 g; 1 hour; 0°C), and centrifuged (12000 rpm; 20 min; 4°C). The resulting precipitate was collected and dissolved in a phosphate buffer (pH 7.8; 20 mM; 0.5 mol NaCl) to give 0.72 mL of the LE solution, named in the following as the solution X2 (Cₚᵣₒₜₑᵢₙ=2980 µg/mL) having the specific activity of 6.62 µmol/min·mg.

### 1c. Purifying the lovastatin esterase by chromatography

A hydrophobic interaction column packed with Phenyl Sepharose 6-fast flow (26 mL) was subjected to equilibration with a phosphate buffer (pH=6.5; 1 mL/min; 5 hours). Then the solution X2 (15 mL) was fed to the column, that was eluted with a phosphate buffer (pH=6.5; 1.4 mL/min), redistilled water (1.4 mL/min), collecting the fractions containing the LE enzyme. Following the separation, the column was rinsed with a phosphate buffer (pH=6.5; 1.4 mL/min; 30 min) again. The active fractions containing the LE enzyme were pooled and the carbonate buffer (1 mL, pH=9.4; 50 mmol) was added to yield 12.5 mL of the solution (Cₚᵣₒₜₑᵢₙ=21 µg/mL) named further the solution X3, having a specific activity of 290 µmol/mg·min (assay by HPLC).

### Example 2. Immobilization of the LE enzyme on Sepharose B4 (agarose gel)

### 2a. Activation of the solid support

Sepharose B4 (5 mL) was washed with water (20 x 5 mL), a phosphate buffer (1/15 M, pH=5.6, 20 x 5 mL), water (20 x 5 mL), a carbonate buffer (50 mmol, pH=9.6, 20 x 5 mL). A 100 mg sample of the obtained bed was dried and analysed.

IR (KBr): 3436 (35%), 2901 (60%), 1650 (55%), 1474 (55%), 1415 (55%), 1376 (50%), 1307 (55%), 1250 (55%), 1190 (35%), 1157 (35%), 1071 (30%), 1042 (%), 988 (55%), 966 (50%), 931 (40%), 891 (45%), 870 (60%), 788 (60%), 772 (55%), 741 (50%), 716 (50%), 693 (50%), 538 (50%), 483 (50%) cm⁻¹.

Elementary analysis: found: C 41.61%; H 6.47%; N 0.0%.

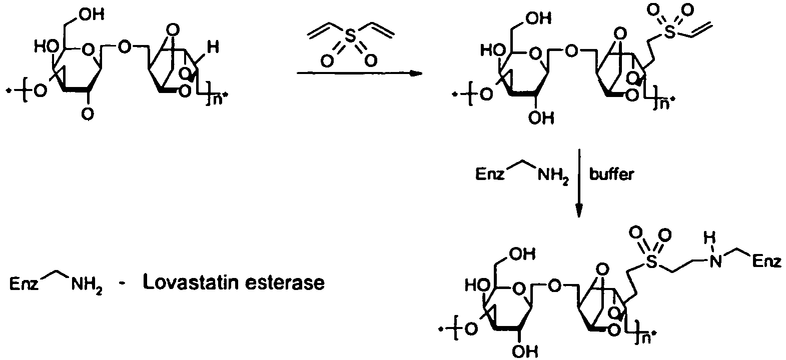

The obtained solid support was suspended in a carbonate buffer (10 mL, 50 mmol pH=9.6), divinylsulphone (1 mL, 10 mmol) was added and the mixture was shaken for 70 min. The solid support was washed with water (20 x 5 mL), a phosphate buffer (20 x 5 mL, 1/15 M, pH=5.6), water (20 x 5 mL), and a carbonate buffer (20 x 5 mL, 50 mmol, pH=9.6). A 100 mg sample of the obtained solid support was dried and analysed.

IR (KBr): 3436 (40%), 2902 (65%), 1651 (55%), 1475 (60%), 1413 (55%), 1377 (50%), 1302 (60%), 1251 (60%), 1183 (40%), 1157 (40%), 1076 (30%), 1044 (30%), 988 (60%), 966 (60%), 931 (50%), 891 (60%), 772 (70%), 741 (70%), 715 (70%) cm⁻¹.

Elementary analysis: found: C 43.50%; H 6.76%; N 0%; S 0.88%.

### 2b. Immobilization of the LE enzyme on a solid support

To the activated solid support, washed with water (20 x 5 mL), a carbonate buffer (5 mL, 50 mM pH=9.6), and the supernatant X1 extracted from the microorganism *Clonostachys compactiuscula,* (Cₚᵣₒₜₑᵢₙ=21178 µg/mL, activity=13.2 µmol/mg·mL) in a carbonate buffer (0.5 mL, 50 mmol, pH=9.6) were added, and the mixture was shaken for 18 hours. The solid support was separated by filtration, suspended in a glycine buffer (10 mL, 50 mmol, pH=9.6), shaken for 2 hours, washed with water (20 x 5 mL), a phosphate buffer (20 x 5 mL, 1/15 mol, pH=5.6), water again (20 x 5 mL), and a glycine buffer (20 x 5 mL, 50 mmol, pH=9.6). 910 µg of the protein was immobilized.

### Example 3. Hydrolysis of a mixture of ammonium salts of the statins with the LE enzyme immobilized on a solid support, in a batch process

The LE enzyme immobilized on a solid support Sepharose B4 (obtained in Example 2b; dry weight of 9.2 mg) was suspended in a mixture of ammonium salts of the statins (1 mL, Cₛₜₐₜᵢₙₛ=0.8 mg/mL) dissolved in a glycine buffer (10 mL, 50 mmol, pH=9.6). The suspension was shaken for 90 minutes at 30°C. The immobilized LE enzyme was separated by filtration, and the hydrolytic activity towards the lovastatin ammonium salt (the hydrolytic activity L) and towards the simvastatin ammonium salt (the hydrolytic activity S), and also the corresponding conversions, were determined by HPLC analysis. The immobilized LE enzyme was washed with water (5 x 5 mL) and the hydrolysis batch process was repeated. After 4 repetitions, 5.7 mg of the immobilized LE enzyme (after drying to a constant weight) were recovered. The results of the analysis for this biocatalyst are presented in Table 1.

**Table 1**

| Repetition | Conversion L (%) | The hydrolytic activity L (µM/mg·mL) | Conversion S (%) | The hydrolytic activity S (µM/g·mL) | Selectivity L/S |
|---|---|---|---|---|---|
| The native LE enzyme | | 10.71 | | 0.06 | >100 |
| 1 | 33.4 | 4.66 | 8.3 | 0.81 | 5.75 |
| 2 | 31.1 | 4.36 | 9.9 | 0.97 | 4.49 |
| 3 | 28.5 | 3.97 | 9.3 | 0.91 | 4.36 |
| 4 | 19.4 | 2.72 | 9.0 | 0.88 | 3.09 |

### Example 4

### Hydrolysis of a mixture of ammonium salts of the statins using the flow reactor

Using 10 mL of Sepharose B4 and the solution X3 of the purified LE enzyme from Example 1c (Cₚᵣₒₜₑᵢₙ=46.1 µg/mL, activity=56.1 nmol/mg·min), immobilization was carried out according to the procedure described in Example 3. 123.1 µg of total protein were immobilized.

The resulting biocatalyst was employed for preparing the flow reactor.

The body of the reactor was a tube of acid-resistant steel (such as used for making high pressure chromatographic columns), containing a perforated plate transverse to the column axis upstream the fluid outlet, that supported the bed. In the Example, a chromatographic column having a diameter of 3.7 mm and a length of 149 mm (inner space volume 1.6 mL) was used, which was protected with a nut and a frit at the bottom, and extended by a ferrule at the top. The above-prepared immobilized enzyme as a suspension in a glycine buffer (pH=9.4, 20 mM, 5 mL) was introduced into this set. The bed was formed by passing the eluent (flow rate: 0.4 mL/min, glycine buffer pH=9.4, 20 mM, 5 mL). Formation of the bed was deemed complete after achieving stabilization of the pressure and stabilization of the absorbance (as determined by an UV detector at λ=254 nm). Then the extending ferrule was removed and the exposed bed was secured with a nut and a frit. The bed in the reactor was subjected to conditioning (flow rate: 0.4 mL/min) by passing a glycine buffer (pH=9.4, 20 mM, 5 mL).

A mixture of the ammonium salts of statins, containing 15% lovastatin and 85% simvastatin, was then introduced into the reactor at a flow rate of 0.4 mL/min. The efflux was sampled every 6 hours and analysed by HPLC. The analysis results, recalculated to the degree of hydrolysis, are summarized in Table 2. During the whole process, the concentration of the simvastatin salt varied not more than by 1%. The lovastatin ammonium salt was hydrolysed in about 87%. This conversion was unchanged over 120 hours.

**Table 2. Hydrolysis of the mixture of statins in a flow reactor with the bed containing the LE enzyme immobilized on a solid support**

| Time of run elapsed before sampling the efflux (hours) | Conversion L (%) | Conversion S (%) | Selectivity (conversion L / conversion S) |
|---|---|---|---|
| 0 | 87.9 | <0.1 | >100 |
| 6 | 86.6 | <0.1 | >100 |
| 12 | 86.1 | <0.1 | >100 |
| 18 | 87.4 | <0.1 | >100 |
| 24 | 87.0 | <0.1 | >100 |
| 30 | 86.8 | <0.1 | >100 |
| 36 | 86.6 | <0.1 | >100 |
| 42 | 86.9 | <0.1 | >100 |
| 48 | 86.9 | <0.1 | >100 |
| 54 | 87.7 | <0.1 | >100 |
| 60 | 87.1 | <0.1 | >100 |
| 66 | 86.6 | <0.1 | >100 |
| 72 | 88.8 | <0.1 | >100 |
| 78 | 88.4 | <0.1 | >100 |
| 84 | 87.9 | <0.1 | >100 |
| 90 | 87.0 | <0.1 | >100 |
| 96 | 90.6 | <0.1 | >100 |
| 102 | 87.2 | <0.1 | >100 |
| 108 | 87.6 | <0.1 | >100 |
| 114 | 86.6 | <0.1 | >100 |
| 120 | 87.6 | <0.1 | >100 |
| 126 | 87.9 | <0.1 | >100 |

### Example 5. Estimation of technological stability of the reactor with the bed containing the LE enzyme immobilized on a solid support

The inlet and the outlet of the flow reactor used in Example 4 were secured with tightly fixed caps, a then the reactor was stored for 6 months at 4°C. After stabilizing, the reactor was used again to hydrolyse the mixture of ammonium salts of statins, following the procedure of Example 4. The results of conversion of the lovastatin salt and the simvastatin salt are summarized in Table 3.

**Table 3. Hydrolysis of the mixture of statins in a flow reactor with the bed containing the LE enzyme immobilized on a solid support (the reactor reused after a 6-month storage at 4°C)**

| Time of run elapsed before sampling the efflux (hours) | Conversion L (%) | Conversion S (%) | Selectivity (conversion L / conversion S) |
|---|---|---|---|
| 0 | 97.1 | <0.1 | >100 |
| 2 | 97.7 | <0.1 | >100 |
| 3 | 98.0 | <0.1 | >100 |
| 5 | 97.6 | <0.1 | >100 |
| 7 | 97.3 | <0.1 | >100 |
| 9 | 97.5 | <0.1 | >100 |
| 11 | 96.6 | <0.1 | >100 |
| 13 | 96.8 | <0.1 | >100 |
| 15 | 96.4 | <0.1 | >100 |
| 17 | 96.2 | <0.1 | >100 |
| 19 | 96.1 | <0.1 | >100 |
| 21 | 96.0 | <0.1 | >100 |
| 23 | 96.7 | <0.1 | >100 |
| 25 | 96.6 | <0.1 | >100 |

### Example 6. Immobilization of the LE enzyme on a solid support (silica gel) using cyanuric chloride

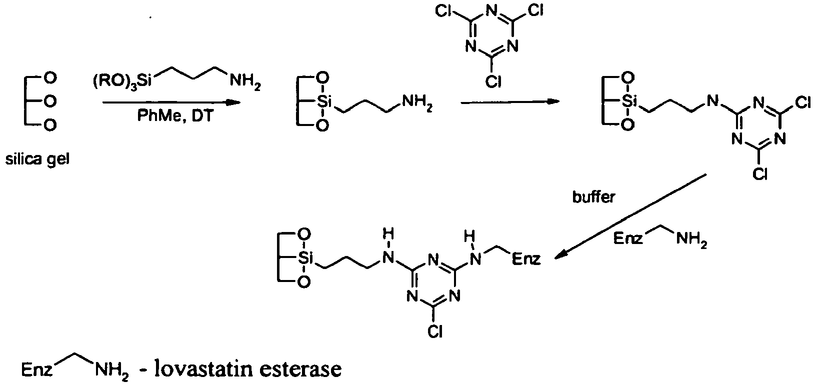

### 6a. Modifying the silica gel

Silica gel (50 g; 200-300 mesh) was placed on a sintered-glass funnel and rinsed with nitric acid (5%, 15 mL) and water (15 mL). A portion of the gel (30 g) was then flooded with toluene (210 mL) and dried by distilling off the azeotropic mixture (water-toluene, 110°C; 4 hours). After cooling to 80°C, (3-aminopropyl)trimethoxysilane (6 mL, 34.3 mmol) was added, and the mixture was heated at 80°C for 2 hours. The obtained solid support was filtered off and rinsed with toluene (20 mL), hexane (20 mL), tetrahydrofuran (20 mL) and toluene again (20 mL). The material was dried at 80°C until the constant weight to yield 24 g of the solid support. Elementary analysis: found: C 4.99%; H 1.12%; N 1.69%.

### 6b. Activation of the solid support and immobilization of the LE enzyme

The aminopropylsilylated silica gel obtained in Example 6a (250 mg, 200-300 mesh, containing 1 mmol of amino groups per 1 g of the solid support) was added to the solution of cyanuric chloride (62.8 mg, 0.34 mmol) in a mixture of dioxane (5 mL) and toluene (1 mL), at 12°C, and shaken on a shaker for 3 hours. The solid support was filtered off, rinsed with toluene (15 mL) and acetone (15 mL), and dried under reduced pressure. The supernatant X1 obtained in Example la (11.5 mL, Cₚᵣₒₜₑᵢₙ=4180 µg/mL) was added to the thus-prepared solid support and shaken on a shaker for 2.5 hours. The obtained biocatalyst (the LE enzyme on a solid support) was filtered off and washed with a carbonate buffer (50 mmol, pH=9.4; 5 x 5 mL) to yield the immobilized enzyme and the filtrate. The immobilization yield was 16%.

The obtained biocatalyst was employed for the hydrolysis of a mixture of ammonium salts of the statins giving a conversion of lovastatin of 7.1% over 90 minutes. The selectivity of the obtained biocatalyst was >100. The hydrolysis reaction carried out using the non-immobilized enzyme (remaining in the filtrate) had a yield of 11.5%.

### Example 7. Immobilization of the LE enzyme on a solid support (modified silica gel) using cyanuric chloride

The aminopropylsilylated silica gel obtained in Example 6a (500 mg, 200-300 mesh, containing 1 mmol of amino groups per 1 g of the bed) was added to the solution of cyanuric chloride (125.9 mg, 0.68 mmol) in a mixture of dioxane (5 mL) and toluene (1 mL), at 9°C, and shaken on a shaker for 3 hours. Then the activated solid support was filtered off, rinsed with toluene (25 mL) and acetone (25 mL), and dried under reduced pressure. A portion of the activated solid support (250 mg) was suspended in the solution X2 obtained in Example 1c (11.5 mL, Cₚᵣₒₜₑᵢₙ=21 µg/mL) and shaken on a shaker for 2.5 hours. The obtained biocatalyst (the LE enzyme on a solid support) was filtered off and washed with a carbonate buffer (50 mmol, pH=9.4, 5 x 5 mL) to yield the immobilized enzyme and the filtrate. The immobilization yield was 52%.

The obtained biocatalyst was employed for the hydrolysis of a mixture of ammonium salts of the statins giving the conversion of lovastatin of 34.1% over 90 minutes. The selectivity of the biocatalyst was >100. The same reaction carried out using the filtrate does not proceed at all, what testifies to complete immobilization of the LE on a solid support.

### Example 8. Immobilization of the LE enzyme on a solid support (modified silica gel) using cyanuric chloride

The aminopropylsilylated silica gel obtained in Example 6a (500 mg, 200-300 mesh, containing I mmol of amino groups per 1 g of the bed) was added to the solution of cyanuric chloride (9.3 mg, 0.05 mmol) in a mixture of dioxane (5 mL) and toluene (1 mL), at 9°C, and shaken on a shaker for 3 hours. The activated solid support was filtered off, rinsed with toluene (25 mL) and acetone (25 mL), and dried under reduced pressure. Then the solid support (250 mg) was suspended in the solution X3 of the LE enzyme obtained in Example 1c (11.5 mL, Cₚᵣₒₜₑᵢₙ=21 µg/mL) and shaken on a shaker for 2.5 hours. The obtained biocatalyst was filtered off and washed with a carbonate buffer (50 mmol, pH=9.4, 5 x 5 mL) to yield the immobilized enzyme and a filtrate. The immobilization yield was 52%.

The obtained biocatalyst was employed for the hydrolysis of a mixture of ammonium salts of the statins, giving the conversion of lovastatin of 16.3% over 90 minutes. The selectivity of the biocatalyst was >100. The same reaction carried out using the filtrate does not proceed at all, what testifies to complete immobilization of the LE on a solid support.

Following the above hydrolysis of a mixture of ammonium salts of the statins, the biocatalyst was rinsed with a carbonate buffer (50 mmol, pH=9.4, 5 x 5 mL) and stored for 7 days at 4°C. Then the biocatalyst was used again for the hydrolysis of a mixture of ammonium salts of the statins giving the conversion of lovastatin of 26.1% over 90 minutes. The selectivity of the biocatalyst was >100.

### Example 9. Immobilization of the LE enzyme on a solid support (diethylaminoethylcellulose) using cyanuric chloride

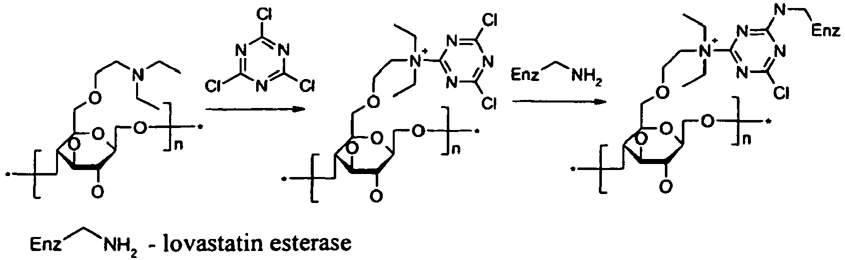

Diethylaminoethylcellulose (0.5 g) was washed with water (10 mL), suspended in a sodium hydroxide solution (1 mol, 10 mL), shaken (15 min, 250 rpm), filtered, washed with water (10 mL) and suspended in dioxane (10 mL). A solution of cyanuric chloride (1 g, 5.4 mmol) in toluene (10 mL) was added to the obtained suspension. The suspension was shaken (30 min, 250 rpm), filtered, the precipitate was rinsed with dioxane (2 x 10 mL), a mixture of acetic acid / water / dioxane (2/2/4 mL), water (10 mL), acetone (2 x 10 mL), and dried under reduced pressure (0.2 tor). The solution X3 obtained in Example 1c (13 mL, specific activity 313 µmol·min⁻¹·mg⁻¹) was added to the activated solid support. The suspension was shaken on a shaker for 18 hours (250 rpm), a then the biocatalyst was filtered off, washed with water (2 x 10 mL), and carbonate buffer (2 x 10 mL, 50 mmol, pH=9.4). The immobilization yield was 37.2%. The activity of the obtained immobilized enzyme was checked by hydrolysing 8 mL of a mixture of ammonium salts of the statins, to obtain the specific activity value of 311 µmol·min⁻¹·mg⁻¹. The selectivity of the biocatalyst was >100.

The above-obtained immobilized LE enzyme was added to the solution of ammonium salts of simvastatin (294 mg, 0.67 mmol) and lovastatin (21.7 mg, 0.05 mmol) in a carbonate buffer (150 mL, 50 mmol, pH=9.4) at 30°C, with magnetic stirring (250 rpm). The reaction progress and selectivity of the hydrolysis are analysed by HPLC.

**Table 4. Conversion of the salt of lovastatin in the reaction catalysed with the LE enzyme immobilized on diethylaminoethylcellulose activated with cyanuric chloride**

| Time of hydrolysis (min) | Conversion (%) | Selectivity L/S |
|---|---|---|
| 0 | 0 | |
| 10 | 11.0 | >100 |
| 15 | 14.9 | >100 |
| 22 | 18.2 | >100 |
| 37 | 30.5 | >100 |
| 57 | 39.6 | >100 |
| 69 | 44.5 | >100 |
| 360 | 100 | >100 |

### Example 10. Immobilization of the LE enzyme on a solid support (diethylaminoethylcellulose) using cyanuric chloride

The procedure described in Example 9 was followed using the previously autoclaved diethylaminoethylcellulose without initial purification and the LE enzyme having the specific activity (L) of 61.2 µmol·min⁻¹·mg⁻¹.

The obtained immobilized LE enzyme was used for the preparative hydrolysis of the mixture of ammonium salts of the statins, according to the procedure described in Example 9.

**Table 5. Conversion of the salt of lovastatin in the reaction catalysed with the LE enzyme immobilized on diethylaminoethylcellulose activated with cyanuric chloride**

| Time of hydrolysis (hours) | Conversion of lovastatin (%) | | Selectivity L/S |
|---|---|---|---|
| | Autoclaved cellulose | Non-autoclaved cellulose | |
| 0 | 0 | 0 | |
| 1.3 | 15.5 | 11.6 | >100 |
| 19.3 | 85.7 | 62.8 | >100 |

It was found that autoclaving of the solid support prior to immobilization of the LE enzyme increased the activity of the biocatalyst by 35%, compared to the enzyme LE immobilized on a non-autoclaved cellulose. The selectivity of the biocatalyst was >100.

### Example 11. Hydrolysis of a mixture of ammonium salts of the statins using the flow reactor

The body of a chromatographic column (0.58 cm in diameter, 9.6 cm length, 2.5 mL inner space volume) was secured with a frit at one end. An extending ferrule was put on the other end. The immobilized LE enzyme obtained in Example 9, as a suspension in a carbonate buffer (10 mL, 50 mmol, pH=9.4, 0.2 mg/mL NaN₃, 0.3 mg/mL EDTA) was introduced into the obtained set. After dribbling the buffer from the frit, the extending ferrule was removed and the exposed bed was secured with the second frit (the amount of the bed corresponded to the weight of product obtained from 0.8 g of diethylaminoethylcellulose and contains 0.62 mg of the immobilized protein). The bed in the reactor was formed with a flow rate of a carbonate buffer (0.4 mL/min, 50 mmol, pH=9.4, 0.2 mg/mL NaN₃, 0.3 mg/mL EDTA) for one hour. Then the solution of the ammonium salts of simvastatin (0.74 mg/mL) and lovastatin (0.06 mg/mL) was pumped through the reactor thermostatted at 28°C. Every 4 hours, the contents of the ammonium salts of the statins in the efflux was determined by HPLC.

The experiments were carried out for the flow rates varying from 0.175 mL/min to 0.2 mL/min.

**Table 6. Effect of the flow rate on the conversion of the lovastatin salt in the reaction catalysed with the LE enzyme immobilized on diethylaminoethylcellulose activated with cyanuric chloride**

| Flow rate (mL/min) | Conversion of lovastatin (%) | Selectivity L/S |
|---|---|---|
| 0.175 | 100 | >100 |
| 0.185 | 92 | >100 |
| 0.2 | 86 | >100 |

By controlling the temperature of the thermostatted column, a thermal dependence of the conversion of lovastatin was determined.

**Table 7. Effect of temperature on the conversion of lovastatin salt in the reaction catalysed with LE enzyme immobilized on diethylaminoethylcellulose activated with cyanuric chloride**

| Temperature (°C) | Conversion of lovastatin (%) | Selectivity L/S |
|---|---|---|
| 28 | 52.1 | >100 |
| 30 | 50.6 | >100 |
| 32 | 50.8 | >100 |
| 34 | 58.0 | >100 |
| 36 | 61.1 | >100 |
| 38 | 59.9 | >100 |
| 40 | 41.3 | >100 |
| 42 | 31.2 | >100 |
| 44 | 20.6 | >100 |

The results presented in Table 7 indicate that the immobilized LE enzyme has the highest activity at 37°C.

### REFERENCE EXAMPLES

### Reference Example 1. Immobilization of the LE enzyme on a modified silica gel

10 g of silica gel were added to the mixture of γ-aminopropyltriethoxysilane and toluene (2% by volume, 100 mL), and the mixture was refluxed for 10 hours. The gel was then filtered off, rinsed with acetone (2 x 5 mL), water (5 mL), acetone again (2 x 5 mL) and dried with dry air for 10 hours. The thus-prepared gel (100 mg) was suspended in a carbonate buffer (2 mL, 50 mM, pH 9.4) and divinylsulphone (0.25 mL, 2.50 mmol) was added at 20°C. After 30 minutes, the solid support was separated off and dried *in vacuo* for 2 hours. This was then rinsed with distilled water (4 x 20 mL), suspended in a carbonate buffer (1 mL, 50 mM, pH 9.4) and soaked with a solution of the LE enzyme (1 mL, 0.225 mg/mL). The mixture was left for 20 hours at 20°C. The solid support with the LE enzyme deposited thereon was filtered off and rinsed with distilled water (2 x 10 mL).

### Reference Example 2. Immobilization of the LE enzyme on wool

Small pieces of wool (50 mg) were suspended in a carbonate buffer (2 mL, 50 mM, pH 9.4) and then divinylsulphone (0.25 mL, 2.5 mmol) was added at 20°C. After 30 minutes, the wool was separated, dried *in vacuo* (2 hours) and rinsed with distilled water (4 x 20 mL). The solid support was suspended in a carbonate buffer (1 mL, 50 mM, pH 9.4) again, soaked with a solution of the LE enzyme (1 mL, 0.225 mg/mL), and left for 10 hours at the room temperature. The solid support with the LE enzyme deposited thereon was filtered off and rinsed with distilled water (2 x 10 mL).

### Reference Example 3. Immobilization of the LE enzyme by a sol-gel method

A solution of the LE enzyme (0.5 mL, 0.225 mg/mL) was mixed with a Tris buffer (0.5 mL, 0.1 M, pH=7.5) and left on a shaker (10 minutes). Then the aqueous solution of polyvinyl alcohol (100 µL, 4% by volume), sodium fluoride (50 µL, 1 M) and isopropyl alcohol (100 µL) were added. After 5 minutes, isobutyltrimethoxysilane (2.5 mmol) and TMOS (0.5 mmol, 74 µL; 76 mg) were added and the mixture was left on a shaker. The mixture was allowed to dry in an open vessel overnight at the room temperature, and then isopropyl alcohol (10 mL) was added. The gel was separated and washed with water (10 mL), isopropyl alcohol, (10 mL) and n-pentane (10 mL). The obtained gel was crushed and left to dry overnight at the room temperature.

### Reference Example 4. Immobilization of the LE enzyme by a sol-gel method

The method according to the Reference Example 3 was used, with a modification consisting in adding Tween^{®} 80 (0.1 mL) to the solution of the enzyme.

### Reference Example 5. Immobilization of the LE enzyme by a sol-gel method

The method according to the Reference Example 3 was used, with a modification consisting in adding the lovastatin ammonium salt (50 mg) to the solution of the enzyme.

### Reference Example 6. Immobilization of the LE enzyme on Eupergit^{®} C

The freeze-dried LE enzyme (17 mg, protein content of 2.6%) was dissolved in a carbonate buffer (1 mL, 50 mM, pH 9.4) and Eupergit^{®} C (50 mg, a copolymer of methacrylamide and glycidyl methacrylate crosslinked with N,N'-methylenebisacrylamide) was added to the solution and left for 1 day at the room temperature. Then immobilized enzyme was filtered off and rinsed with distilled water (2 x 10 mL).

### Reference Example 7. Immobilization of the LE enzyme by encapsulation in calcium alginate

A solution of the purified LE enzyme (0.8 mL, 0.225 mg/mL) was mixed thoroughly with an aqueous solution of calcium alginate (8 mL, 2% wt./v.). Then, the thus-obtained solution was added dropwise to the aqueous solution of calcium chloride (10 mL, 280 mM), mixed for 20 minutes, the precipitate was filtered off, and washed with a distilled water (2 x 10 mL). The obtained immobilizate was stored under distilled water at 4°C.

### Reference Example 8. Immobilization of the LE enzyme by encapsulation in calcium alginate

A solution of the purified enzyme (0.8 mL, 0.225 mg/mL) was mixed thoroughly with an aqueous solution of calcium alginate (8 mL, 2% wt./v.). Then, the thus-obtained solution was added dropwise to the aqueous solution of calcium chloride (10 mL, 280 mM), mixed for 20 minutes, the precipitate was filtered off, washed with a distilled water (2 x 10 mL). The obtained material was suspended in tetramethoxysilane (1.0 mL) and stirred for 15 minutes at 4°C. The whole mixture was allowed to polymerize for 12 hours. The immobilized enzyme was filtered off, washed with a distilled water (2 x 10 mL) and dried at the room temperature.

### Reference Example 9

The immobilized LE enzymes obtained in the Reference Examples 1-8 were added to the solution of the ammonium salts of simvastatin (294 mg, 0.67 mmol) and lovastatin (21.7 mg, 0.05 mmol) in a carbonate buffer (150 mL, 50 mmol, pH=9.4) at 30°C, with magnetic stirring (250 rpm). The reaction progress and the selectivity of the hydrolysis were analysed by HPLC. The results obtained for the materials prepared in Reference Examples 1-8 are presented in Table 8.

**Table 8**

| Reference Example | Yield of immobilization (%)^{a} | Specific LE activity^{b} | | Selectivity^{c} |
|---|---|---|---|---|
| | | Lovastatin | Simvastatin | |
| The native LE enzyme | - | 15 | 0 | >99 |
| 1 | 80 | <0.5 | <0.5 | N.D. |
| 2 | 54 | <0.5 | <0.5 | N.D. |
| 3 | 74 | 0.9 | 3.0 | 0.3 |
| 4 | 67 | 10 | 17.8 | 0.6 |
| 5 | 61 | 7.4 | 6.2 | 1.2 |
| 6 | 80 | 8.5 | 4.2 | 2.0 |
| 7 | N.D. | N.D. | N.D. | 3.5 |
| 8 | N.D. | <0.5 | <0.5 | N.D. |

| | | | | |
|---|---|---|---|---|
| N.D. - not determined ^{a)} the yield of immobilization is determined as: an amount of the immobilized protein / total amount of the proteins 100%; ^{b)} the amount of the respective substrate hydrolyzed with the enzyme within one minute per milligram of protein (µM/min·mg); ^{c)} the specific LE activity for lovastatin / the specific activity LE for simvastatin. | | | | |

## Claims

1. The lovastatin esterase enzyme immobilized on a solid support insoluble in water, said enzyme being covalently bound to a solid support activated with an at least difunctional coupling reagent, **characterized in that** the combination of solid support and at least difunctional coupling reagent is such, that the immobilized lovastatin esterase exhibits at least 5 times higher the hydrolytic activity towards lovastatin and salts thereof, in the presence of simvastatin and/or salts thereof, than towards simvastatin and salts thereof, wherein:
i) the solid support is a modified polysaccharide comprising di-(C₁₋₆alkyl)amino-C₁₋₆alkylcellulose, and the at least difunctional reagent activating the solid support is cyanuric acid O-sulphonate or cyanuric halide, or
ii) the solid support is a modified silica gel, especially modified with amino-C₁₋₆ alkyl-tri(C₁₋₆ alkoxy)silane, and the at least difunctional reagent activating the solid support is cyanuric acid O-sulphonate or cyanuric halide, or
iii) the solid support is a polygalactoside and the at least difunctional reagent activating the solid support is a compound of the formula , in which Y represents -SO₂- or -SO₂-(CHR)ₙ-SO₂-, where n is an integer of from 1 to 18, and R is a hydrogen atom or C₁₋₆ alkyl, or Y represents -SO₂-Ar-SO₂-, where Ar is a divalent aryl radical formed by displacing two hydrogen atoms directly bound to the aromatic ring carbon atoms, the divalent aryl radical optionally bearing C₁₋₆ alkyl substituents.

2. The lovastatin esterase enzyme according to claim 1, wherein the solid support is diethylaminoethylcellulose, and the at least difunctional reagent activating the solid support is cyanuric chloride.

3. The lovastatin esterase enzyme according to claim 1, wherein the solid support is an aminopropylsilanized silica gel, and the at least difunctional reagent activating the solid support is cyanuric chloride.

4. The lovastatin esterase enzyme according to claim 1, wherein the solid support is a polygalactoside, and the at least difunctional reagent activating the solid support is the compound of the formula 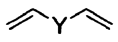 , in which Y represents -SO₂-.

5. The lovastatin esterase enzyme according to claim 1, 2, 3 or 4, **characterized in that** the enzyme is an enzyme produced by *Clonostachys compactiuscula* ATCC 38009, ATCC 74178.

6. A process for immobilization of the lovastatin esterase enzyme on a solid support insoluble in water, **characterized in that** using mechanical agitation, in a solvent, a cyanuric halide is contacted with a solid support being modified polysaccharide comprising di-(C₁₋₆alkyl)amino-C₁₋₆alkylcellulose or a solid support being silica gel modified with amino-C₁₋₆alkyl-tri(C₁₋₆alkoxy)silane, the activated solid support is separated by filtration, then dried and suspended in an aqueous mixture containing the lovastatin esterase enzyme until immobilization of the enzyme, the suspended material is separated by filtration, washed with a buffer and dried.

7. A process according to claim 6, wherein the solid support is diethylaminoethylcellulose.

8. A process according to claim 6, wherein the solid support is aminopropylsilanized silica gel.

9. A process according to claim 6, wherein cyanuric halide used is cyanuric chloride.

10. A process according to claim 7, 8 or 9, wherein an autoclaved solid support is used.

11. A process according to claim 6, wherein the enzyme-containing aqueous solution used is a protein fraction of the material extracted from *Clonostachys compactiuscula* ATCC 38009, ATCC 74178.

12. A process for immobilization of the lovastatin esterase enzyme on a solid support insoluble in water, **characterized in that** the compound of the formula 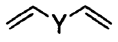 , in which Y represents -SO₂- or -SO₂-(CHR)ₙ-SO₂-, where n is an integer of from 1 to 18, and R is a hydrogen atom or C₁₋₆ alkyl, or Y represents -SO₂-Ar-SO₂-, where Ar is a divalent aryl radical formed by displacing two hydrogen atoms directly bound to the aromatic ring carbon atoms, the divalent aryl radical optionally bearing C₁₋₆ alkyl substituents, is contacted with the solid polygalactose support in a solvent using mechanical agitation, the activated solid support is separated by filtration, then dried and suspended in an aqueous mixture containing the lovastatin esterase enzyme until immobilization of the enzyme, the suspended material is separated by filtration, washed with a buffer and dried.

13. A process according to claim 12, wherein the compound of the formula 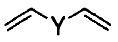 is a compound in which Y represents -SO₂-.

14. A process according to claim 12, wherein the enzyme-containing aqueous solution used is a protein fraction of the material extracted from *Clonostachys compactiuscula* ATCC 38009, ATCC 74178.

15. A biocatalytic flow reactor with a bed comprising a body (1) of the reactor with an inner space (2) connected to the fluid inlet (3) and connected to the fluid outlet (4), in which inner space (2) there is a bed (5) containing the lovastatin esterase enzyme immobilized on a solid support insoluble in water, said enzyme being covalently bound to the solid support activated with an at least difunctional coupling reagent, **characterized in that** the combination of solid support and at least difunctional coupling reagent is such, that the immobilized lovastatin esterase exhibits at least 5 times higher the hydrolytic activity towards lovastatin and salts thereof in the presence of simvastatin and/or salts thereof, than towards simvastatin and salts thereof,
wherein:
i) the solid support is a polygalactoside, and the at least difunctional reagent activating the solid support is a compound of the formula 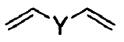 , in which Y represents -SO₂-, or
ii) the solid support is diethylaminoethylcellulose, and the at least difunctional reagent activating the solid support is cyanuric chloride.

16. A biocatalytic flow reactor according to claim 15, wherein the enzyme is an enzyme produced by *Clonostachys compactiuscula* ATCC 38009, ATCC 74178.

17. A process for preparation and/or purification of simvastatin comprising treating the solution of the simvastatin salt containing residual content of the lovastatin salt with the lovastatin esterase enzyme until hydrolysing lovastatin to form the triol, separating the triol, and isolating simvastatin substantially free from lovastatin, where the solution of the simvastatin salt containing residual content of the lovastatin salt is brought into a contact with the lovastatin esterase enzyme immobilized on a solid support insoluble in water, said enzyme being covalently bound to the solid support activated with an at least difunctional coupling reagent, **characterized in that** the combination of solid support and at least difunctional coupling reagent is such, that the immobilized lovastatin esterase exhibits at least 5 times higher the hydrolytic activity towards lovastatin and salts thereof in the presence of simvastatin and/or salts thereof, than towards simvastatin and salts thereof,
wherein:
i) the solid support is a polygalactoside, and the at least difunctional reagent activating the solid support is the compound of the formula , in which Y represents -SO₂-, or
ii) the solid support is diethylaminoethylcellulose, and the at least difunctional reagent activating the solid support is cyanuric chloride, or
iii) the solid support is an aminopropylsilanized silica gel, and the at least difunctional reagent activating the solid support is cyanuric chloride.

18. A process for preparation and/or purification of simvastatin according to claim 17, wherein the enzyme is an enzyme produced by *Clonostachys compactiuscula* ATCC 38009, ATCC 74178.

## Patentansprüche

1. Enzym Lovastatin-Esterase, das auf einem wasserunlöslichen festen Träger immobilisiert ist, wobei das Enzym kovalent an einen festen Träger gebunden ist, der mit einem zumindest difunktionellen Kopplungsreagenz aktiviert ist,
**dadurch gekennzeichnet, dass** die Kombination von festem Träger und zumindest difunktionellem Kopplungsreagenz derart ist, dass die immobilisierte Lovastatin-Esterase in Gegenwart von Simvastatin und/oder Salzen davon eine mindestens 5 Mal höhere hydrolytische Aktivität gegenüber Lovastatin und dessen Salzen als gegenüber Simvastatin und dessen Salzen zeigt,
wobei:
i) der feste Träger ein modifiziertes Polysaccharid ist, das Di-(C₁₋₆-alkyl)amino-C₁₋₆-alkylcellulose aufweist, und das zumindest difunktionelle Reagenz, das den festen Träger aktiviert, Cyanursäure-O-sulfonat oder Cyanurhalogenid ist, oder
ii) der feste Träger ein modifiziertes, insbesondere mit Amino-C₁₋₆-alkyl-tri(C₁₋₆-alkoxy)silan modifiziertes, Kieselgel ist und das zumindest difunktionelle Reagenz, das den festen Träger aktiviert, Cyanursäure-O-sulfonat oder Cyanurhalogenid ist, oder
iii) der feste Träger ein Polygalactosid ist und das zumindest difunktionelle Reagenz, das den festen Träger aktiviert, eine Verbindung mit der Formel 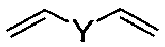 ist, worin Y für -SO₂- oder -SO₂-(CHR)ₙ-SO₂- steht, wobei n eine ganze Zahl von 1 bis 18 ist und R ein Wasserstoffatom oder C₁₋₆-Alkyl ist, oder Y für -SO₂-Ar-SO₂- steht, wobei Ar ein zweiwertiger Arylrest ist, der durch Umlagern von zwei Wasserstoffatomen, die direkt an die Kohlenstoffatome des aromatischen Rings gebunden sind, erzeugt wird, wobei der zweiwertige Arylrest gegebenenfalls C₁₋₆-Alkyl-Substituenten aufweist.

2. Enzym Lovastatin-Esterase nach Anspruch 1,
wobei der feste Träger Diethylaminoethylcellulose ist und das zumindest difunktionelle Reagenz, das den festen Träger aktiviert, Cyanurchlorid ist.

3. Enzym Lovastatin-Esterase nach Anspruch 1,
wobei der feste Träger ein aminopropylsilanisiertes Kieselgel ist und das zumindest difunktionelle Reagenz, das den festen Träger aktiviert, Cyanurchlorid ist.

4. Enzym Lovastatin-Esterase nach Anspruch 1,
wobei der feste Träger ein Polygalactosid ist und das zumindest difunktionelle Reagenz, das den festen Träger aktiviert, eine Verbindung der Formel 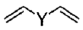 ist, worin Y für -SO₂- steht.

5. Enzym Lovastatin-Esterase nach Anspruch 1, 2, 3 oder 4,
**dadurch gekennzeichnet, dass** das Enzym ein Enzym ist, das von *Clonostachys compactiuscula* ATCC 38009, ATCC 74178 produziert wird.

6. Verfahren zum Immobilisieren des Enzyms Lovastatin-Esterase auf einem wasserunlöslichen festen Träger,
**dadurch gekennzeichnet, dass** unter Anwendung des mechanischen Rührens in einem Lösungsmittel ein Cyanurhalogenid mit einem festen Träger, der modifiziertes Polysaccharid ist, das Di-(C₁₋₆-alkyl)amino-C₁₋₆-alkylcellulose aufweist, oder mit einem festen Träger in Kontakt gebracht wird, der Kieselgel ist, das mit Amino-C₁₋₆-alkyl-tri(C₁₋₆-alkoxy)silan modifiziert ist, wobei der aktivierte feste Träger durch Filtration abgetrennt, danach getrocknet und in einem wässrigen Gemisch, dass das Enzym Lovastatin-Esterase enthält, bis zur Immobilisierung des Enzym suspendiert wird, das suspendierte Material durch Filtration abgetrennt, mit einem Puffer gewaschen und getrocknet wird.

7. Verfahren nach Anspruch 6,
wobei der feste Träger Diethylaminoethylcellulose ist.

8. Verfahren nach Anspruch 6,
wobei der feste Träger aminopropylsilanisiertes Kieselgel ist.

9. Verfahren nach Anspruch 6,
wobei das verwendete Cyanurhalogenid Cyanurchlorid ist.

10. Verfahren nach Anspruch 7, 8 oder 9,
wobei ein einer Autoklavenbehandlung unterzogener fester Träger verwendet wird.

11. Verfahren nach Anspruch 6,
wobei die verwendete, Enzym enthaltende, wässrige Lösung eine Proteinfraktion des Materials ist, das aus *Clonostachys compactiuscula* ATCC 38009, ATCC 74178 herausgelöst worden ist.

12. Verfahren zum Immobilisieren des Enzyms Lovastatin-Esterase auf einem wasserunlöslichen festen Träger
**dadurch gekennzeichnet, dass** die Verbindung der Formel 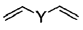 , worin Y für -SO₂- oder -SO₂-(CHR)ₙ-SO₂- steht, wobei n eine ganze Zahl von 1 bis 18 ist und R ein Wasserstoffatom oder C₁₋₆-Alkyl ist, oder Y für -SO₂-Ar-SO₂- steht, wobei Ar ein zweiwertiger Arylrest ist, der durch Umlagern von zwei Wasserstoffatomen, die direkt an die Kohlenstoffatome des aromatischen Rings gebunden sind, erzeugt wird,
wobei der zweiwertige Arylrest gegebenenfalls C₁₋₆-Alkylsubstituenten aufweist, unter Anwendung des mechanischen Rührens in einem Lösungsmittel mit dem festen Polygalactoseträger in Kontakt gebracht wird, der aktivierte feste Träger durch Filtration abgetrennt, danach getrocknet und in einem wässrigen Gemisch, das das Enzym Lovastatin-Esterase enthält, bis zur Immobilisierung des Enzyms suspendiert wird, das suspendierte Material durch Filtration abgetrennt, mit einem Puffer gewaschen und getrocknet wird.

13. Verfahren nach Anspruch 12,
wobei die Verbindung der Formel 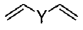 eine Verbindung ist, worin Y für -SO₂-steht.

14. Verfahren nach Anspruch 12,
wobei die verwendete, Enzym enthaltende, wässrige Lösung eine Proteinfraktion des Materials ist, das aus *Clonostachys compactiuscula* ATCC 38009, ATCC 74178 herausgelöst worden ist.

15. Biokatalytischer Durchflussreaktor mit einem Bett, der einen Grundkörper (1) des Reaktors mit einem Innenraum (2) aufweist, der mit dem Fluideinlass (3) verbunden ist und mit dem Fluidauslass (4) verbunden ist, wobei sich im Innenraum (2) ein Bett (5) befindet, das das Enzym Lovastatin-Esterasse enthält, das auf einem wasserunlöslichen festen Träger immobilisiert ist, wobei das Enzym kovalent an den festen Träger gebunden ist, der mit einem zumindest difunktionellen Kopplungsreagenz aktiviert ist,
**dadurch gekennzeichnet, dass** die Kombination von festem Träger und zumindest difunktionellem Kopplungsreagenz derart ist, dass die immobilisierte Lovastatin-Esterase in Gegenwart von Simvastatin und/oder Salzen davon eine mindestens 5 Mal höhere hydrolytische Aktivität gegenüber Lovastatin und dessen Salzen als gegenüber Simvastatin und dessen Salzen zeigt,
wobei:
i) der feste Träger ein Polygalactosid ist und das zumindest difunktionelle Reagenz, das den festen Träger aktiviert, eine Verbindung mit der Formel 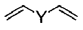 ist, worin Y für -SO₂- steht, oder
ii) der feste Träger Diethylaminoethylcellulose ist und das zumindest difunktionelle Reagenz, das den festen Träger aktiviert, Cyanurchlorid ist.

16. Biokatalytischer Durchflussreaktor nach Anspruch 15,
wobei das Enzym ein Enzym ist, das von *Clonostachys compactiuscula* ATCC 38009, ATCC 74178 produziert wird.

17. Verfahren zum Herstellen und/oder Reinigen von Simvastatin, das Folgendes aufweist:
Behandeln der Simvastatinsalzlösung, die einen Restgehalt an Lovastatinsalz aufweist, mit dem Enzym Lovastatin-Esterase, bis Lovastatin hydrolysiert, wodurch das Triol erzeugt wird, Abtrennen des Triols und Isolieren von Simvastatin, das im Wesentlichen frei von Lovastatin ist, wobei die Simvastatinsalzlösung, die einen restlichen Gehalt an Lovastatinsalz aufweist, mit dem Enzym Lovastatin-Esterase in Kontakt gebracht wird, dass auf einem wasserunlöslichen festen Träger immobilisiert ist, wobei das Enzym kovalent an den festen Träger gebunden ist, der mit einem zumindest difunktionellen Kopplungsreagenz aktiviert ist,
**dadurch gekennzeichnet, dass** die Kombination von festem Träger und zumindest difunktionellem Kopplungsreagenz derart ist, dass die immobilisierte Lovastatin-Esterase in Gegenwart von Simvastatin und/oder Salzen davon eine mindestens 5 Mal höhere hydrolytische Aktivität gegenüber Lovastatin und dessen Salzen als gegenüber Simvastatin und dessen Salzen zeigt,
wobei:
i) der feste Träger ein Polygalactosid ist und das zumindest difunktionelle Reagenz, das den festen Träger aktiviert, eine Verbindung mit der Formel 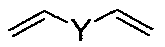 ist, worin Y für -SO₂- steht, oder
ii) der feste Träger Diethylaminoethylcellulose ist und das zumindest difunktionelle Reagenz, das den festen Träger aktiviert, Cyanurchlorid ist, oder
iii) der feste Träger ein aminopropylsilanisiertes Kieselgel ist und das zumindest difunktionelle Reagenz, das den festen Träger aktiviert, Cyanurchlorid ist.

18. Verfahren zum Herstellen und/oder Reinigen von Simvastatin nach Anspruch 17, wobei, das Enzym ein Enzym ist, das von *Clonostachys compactiuscula* ATCC 38009, ATCC 74178 produziert wird.

## Revendications

1. Enzyme lovastatine estérase immobilisée sur un support solide insoluble dans l'eau, ladite enzyme étant liée de manière covalente à un support solide activé par un réactif de couplage au moins difonctionnel, **caractérisé en ce que** la combinaison de support solide et de réactif de couplage au moins difonctionnel est telle, que la lovastatine estérase immobilisée présente une activité hydrolytique au moins 5 fois supérieure envers la lovastatine et des sels de celle-ci, en présence de simvastatine et/ou des sels de celle-ci, qu'envers la simvastatine et des sels de celle-ci,
dans lequel:
i) le support solide est un polysaccharide modifié comprenant une di-(C₁₋₆alkyle)amino-C₁₋₆alkylcellulose, et le réactif au moins difonctionnel activant le support solide est un acide cyanurique O-sulfonate ou un halogénure cyanurique, ou
ii) le support solide est un gel de silice modifié, spécialement modifié avec un amino-C₁₋₆akyl-tri(C₁₋₆alkoxy)silane, et le réactif au moins difonctionnel activant le support solide est un acide cyanurique O-sulfonate ou un halogénure cyanurique, ou
iii) le support solide est un polygalactoside et le réactif au moins difonctionnel activant le support solide est un composé de formule 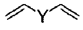 , dans lequel Y représente -SO₂- ou -SO₂-(CHR)ₙ-SO₂-, où n est un entier de 1 à 18, et R est un atome d'hydrogène ou un alkyle C₁₋₆, ou Y représente -SO₂-Ar-SO₂-, où Ar est un radical aryle divalent formé par déplacement de deux atomes d'hydrogène directement liés aux atomes de carbone du cycle aromatique, le radical aryle divalent portant facultativement des substituants alkyles C₁₋₆.

2. Enzyme lovastatine estérase selon la revendication 1, dans laquelle le support solide est une diéthylaminoéthylcellulose, et le réactif au moins difonctionnel activant le support solide est un chlorure cyanurique.

3. Enzyme lovastatine estérase selon la revendication 1, dans laquelle le support solide est un gel de silice aminopropylsilanisé, et le réactif au moins difonctionnel activant le support solide est un chlorure cyanurique.

4. Enzyme lovastatine estérase selon la revendication 1, dans laquelle le support solide est un polygalactoside, et le réactif au moins difonctionnel activant le support solide est le composé de formule 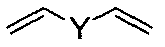 , dans lequel Y représente -SO₂-.

5. Enzyme lovastatine estérase selon la revendication 1, 2, 3 ou 4, **caractérisée en ce que** l'enzyme est une enzyme produite par *Clonostachys compactiuscula* ATCC 38009, ATCC 74178.

6. Procédé pour immobilisation de l'enzyme lovastatine estérase sur un support solide insoluble dans l'eau, **caractérisé par** l'utilisation d'une agitation mécanique, dans un solvant, un halogénure cyanurique est en contact avec un support solide étant un polysaccharide modifié comprenant un di-(C₁₋₆alkyle)amino-C₁₋₆alkylcellulose ou un support solide étant un gel de silice modifié avec une amino-C₁₋₆alkyl-tri(C₁₋₆alkoxy)silane, le support solide activé est séparé par filtration, puis séché et suspendu dans un mélange aqueux contenant l'enzyme lovastatine estérase jusqu'à immobilisation de enzyme, la matière suspendue est séparée par filtration, lavée avec un tampon et séchée.

7. Procédé selon la revendication 6, dans lequel le support solide est une diéthylaminoéthylcellulose.

8. Procédé selon la revendication 6, dans lequel le support solide est un gel de silice aminopropylsilanisé.

9. Procédé selon la revendication 6, dans lequel l'halogénure cyanurique utilisé est un chlorure cyanurique.

10. Procédé selon la revendication 7, 8 ou 9, dans lequel un support solide autoclavé est utilisé.

11. Procédé selon la revendication 6, dans lequel la solution aqueuse contenant une enzyme utilisée est une fraction protéique de la matière extraite du *Clonostachys compactiuscula* ATCC 38009, ATCC 74178.

12. Procédé pour immobilisation de l'enzyme lovastatine estérase sur un support solide insoluble dans l'eau, **caractérisé en ce que** le composé de formule 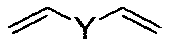 , dans lequel Y représente -SO₂- ou -SO₂-(CHR)ₙ-SO₂-, où n est un entier de 1 à 18, et R est un atome d'hydrogène ou un alkyle C₁₋₆, ou Y représente -SO₂-Ar-SO₂-, où Ar est un radical aryle divalent formé par déplacement de deux atomes d'hydrogène directement liés aux atomes de carbone du cycle aromatique, le radical aryle divalent portant facultativement des substituants alkyles C₁₋₆, est en contact avec le support solide en polygalactose dans un solvant en utilisant une agitation mécanique, le support solide activé est séparé par filtration, puis séché et suspendu dans un mélange aqueux contenant l'enzyme lovastatine estérase jusqu'à immobilisation de l'enzyme, la matière suspendue est séparée par filtration, lavée avec un tampon et séchée.

13. Procédé selon la revendication 12, dans lequel le composé de formule 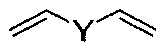 est un composé dans lequel Y représente -SO₂-.

14. Procédé selon la revendication 12, dans lequel la solution aqueuse contenant une enzyme utilisée est une fraction protéique de la matière extraite de *Clonostachys compactiuscula* ATCC 38009, ATCC 74178.

15. Réacteur biocatalytique à circulation avec un lit comprenant un corps (1) du réacteur avec un espace interne (2) connecté à l'entrée de fluide (3) et connecté à la sortie de fluide (4), dans lequel dans l'espace interne (2) duquel il y a un lit (5) contenant l'enzyme lovastatine estérase immobilisée sur un support solide insoluble dans l'eau, ladite enzyme étant liée de manière covalente au support solide activé avec un réactif de couplage au moins difonctionnel, **caractérisé en ce que** la combinaison de support solide et de réactif de couplage au moins difonctionnel est telle, que la lovastatine estérase immobilisée présente une activité hydrolytique au moins 5 fois supérieure envers la lovastatine et des sels de celle-ci en présence de simvastatine et/ou des sels de celle-ci, qu'envers la simvastatine et des sels de celle-ci,
dans lequel:
i) le support solide est un polygalactoside, et le réactif au moins difonctionnel activant le support solide est un composé de formule 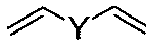 , dans lequel Y représente -SO₂-, ou
ii) le support solide est une diéthylaminoéthylcellulose, et le réactif au moins difonctionnel activant le support solide est un chlorure cyanurique.

16. Réacteur biocatalytique à circulation selon la revendication 15, dans lequel l'enzyme est une enzyme produite par *Clonostachys compactiuscula* ATCC 38009, ATCC 74178.

17. Procédé pour la préparation et/ou la purification de simvastatine comprenant traitement de la solution de sel de simvastatine contenant une teneur résiduelle de sel de lovastatine avec l'enzyme lovastatine estérase jusqu'à hydrolyse de la lovastatine pour former le triol, séparation du triol, et isolation de la simvastatine substantiellement exempte de lovastatine, où la solution de sel de simvastatine contenant une teneur résiduelle de sel de lovastatine est mise en contact avec l'enzyme lovastatine estérase immobilisée sur un support solide insoluble dans l'eau, ladite enzyme étant liée de manière covalente au support solide activé avec un réactif de couplage au moins difonctionnel, **caractérisé en ce que** la combinaison de support solide et de réactif de couplage au moins difonctionnel est telle, que la lovastatine estérase immobilisée présente une activité hydrolytique envers la lovastatine et des sels de celle-ci au moins 5 fois supérieure en présence de simvastatine et/ou des sels de celle-ci, qu'envers la simvastatine et des sels de celle-ci,
dans lequel:
i) le support solide est un polygalactoside, et le réactif au moins difonctionnel activant le support solide est le composé de formule 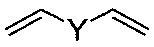 dans lequel Y représente -SO₂-, ou
ii) le support solide est une diéthylaminoéthylcellulose, et le réactif au moins difonctionnel activant le support solide est un chlorure cyanurique, ou
iii) le support solide est un gel de silice aminopropylsilanisé, et le réactif au moins difonctionnel activant le support solide est un chlorure cyanurique.

18. Procédé pour la préparation et/ou la purification de simvastatine selon la revendication 17, dans lequel enzyme est une enzyme produite par *Clonostachys compactiuscula* ATCC 38009, ATCC 74178.
